# EUROPEAN PATENT APPLICATION

(11) **EP 4 759 297 A2**
(43) Date of publication of application: **17.06.2026**
(21) Application number: 26170627.9
(22) Date of filing: 02.04.2026
(51) Int. Cl.: A61K 8/34, A61K 8/42, A61K 8/49, A61P 17/10, A61Q 11/00, A61Q 15/00, A61Q 17/00

(54) **USE OF COMPOUNDS FOR THE INHIBITION OF OBLIGATE ANAEROBIC BACTERIA**

(71) Applicant: CLARIANT INTERNATIONAL LTD, 4132 Muttenz (CH)
(72) Inventor: The designation of the inventor has not yet been filed
(74) Representative: Zusammenschluss Clariant

(57) **Abstract**

The present invention relates to the use of amphiphilic compounds as antimicrobial agents specifically effective against obligate anaerobic bacteria under strict anaerobic conditions. The compounds include glyceryl ethers, glyceryl esters, sorbitan esters, isosorbide esters, and N-methyl cyclic glucamides of formula (I): 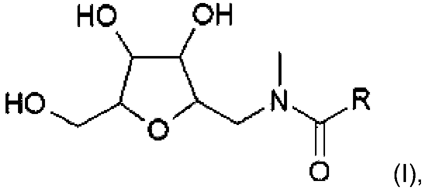 wherein R is a saturated or unsaturated hydrocarbon chain having 5 to 23 carbon atoms. The compounds demonstrate antimicrobial activity against clinically relevant obligate anaerobic bacteria when tested under strict anaerobic conditions that accurately reflect the natural environments where these bacteria thrive. The invention provides compounds for therapeutic and non-therapeutic uses targeting obligate anaerobic bacteria, as well as antimicrobial compositions suitable for personal care, cosmetic, and pharmaceutical applications.

## Description

### BACKGROUND OF THE INVENTION

The present invention relates to the use of certain compounds as antimicrobial active substances against obligate anaerobic bacteria, particularly glyceryl ethers, glyceryl esters, sorbitan esters, isosorbide esters, and N-methyl cyclic glucamides. The invention further relates to compounds for use in treating medical conditions caused by obligate anaerobic bacteria, to compositions for inhibiting obligate anaerobic bacteria, and to *in vitro* and *ex vivo* methods for inhibiting obligate anaerobic bacteria.

Antimicrobial compounds are widely used in personal care, cosmetic, and pharmaceutical formulations to prevent microbial contamination, preserve product integrity, and provide therapeutic benefits. Various classes of compounds have been investigated for their antimicrobial properties, including esters of polyols such as sorbitan, isosorbide, and glycerol, as well as N-alkylated sugar derivatives.

Various polyol esters have been disclosed as antimicrobial agents. U.S. Patent No. 3,331,742 discloses fatty acid esters of polyhydric alcohols as emulsifiers and antimicrobial agents. JP 8187070 A discloses mixtures of fatty acid esters of polyols including sorbitol, sorbitan, and isosorbide as antimicrobially active compounds. DE 10 2009 022 444 A1 and DE 10 2009 022 445 A1 describe compositions comprising sorbitan monocaprylate for preserving cosmetic, dermatological, or pharmaceutical products. These prior art documents focus on preservation against aerobic microorganisms and fungi, without addressing anaerobic bacteria.

U.S. Patent No. 9,730,450 B2 discloses isosorbide monoesters as antimicrobial active substances, particularly as fungicides. The patent describes efficacy against yeasts such as *Candida albicans* and fungi, as well as aerobic bacteria including *Staphylococcus aureus, Pseudomonas aeruginosa,* and *Escherichia coli.* The patent does not disclose testing under strict anaerobic conditions or provide data on activity against anaerobic bacteria.

U.S. Patent Application Publication No. 2014/0322151 A1 describes the use of isosorbide caprylates and caprates in deodorants and antiperspirants for reducing body odor. The patent focuses on deodorizing effects rather than antimicrobial testing. No specific bacteria are tested, and there is no disclosure of testing under anaerobic conditions or activity against obligate anaerobic bacteria under such conditions.

WO 2018/002100 describes N-methyl cyclic glucamides of formula (I) and their synthesis, but does not specifically address activity against obligate anaerobic bacteria under anaerobic conditions.

WO 2024/194297 A1 discloses the use of N-methyl cyclic glucamides (such as Capryloyl/Caproyl Anhydro Methyl Glucamide), glyceryl ethers, sorbitan esters, and isosorbide esters as antimicrobial agents against oral microorganisms. The patent focuses on *Streptococcus mutans* and *Lactobacillus acidophilus* as preferred target bacteria, with all experimental testing conducted exclusively on S. *mutans* using standard aerobic protocols (BS EN 1040:2005). The patent does not disclose testing under strict anaerobic conditions or provide MIC data for obligate anaerobic bacteria.

WO 2025/058767 A1 describes antimicrobial compositions comprising N-methyl cyclic glucamides for use in deodorants and antiperspirants. While antimicrobial activity is claimed, the patent does not specifically address obligate anaerobic bacteria or provide data on activity against such organisms under anaerobic conditions.

The prior art documents described above share common limitations. None describe antimicrobial testing conducted under strict anaerobic conditions suitable for obligate anaerobic bacteria. The prior art focuses primarily on aerobic bacteria, fungi, and yeasts, and does not provide MIC values determined under anaerobic conditions for obligate anaerobic bacteria. Furthermore, the prior art does not recognize or exploit the specific activity of these compounds against obligate anaerobic bacteria for applications such as treating skin conditions associated with anaerobic bacteria, controlling malodor caused by anaerobic bacteria on the skin, or targeting obligate anaerobes in periodontal pockets.

Obligate anaerobic bacteria are microorganisms that cannot survive in the presence of oxygen and require anaerobic conditions for growth. These bacteria play significant roles in various human health conditions. Examples of clinically relevant obligate anaerobic bacteria include, but are not limited to, bacteria of the *Cutibacterium sp., Fusobacterium sp., Anaerococcus sp., Bacteroides sp., Clostridioides sp., Porphyromonas sp., Prevotella sp.* and *Peptostreptococcus sp..* Obligate anaerobic bacteria colonize low-oxygen or oxygen-free environments in the human body, such as sebaceous follicles, periodontal pockets, and skin surfaces. Certain obligate anaerobes are associated with skin conditions such as acne, and oral conditions such as periodontal disease and halitosis.

Effective antimicrobial agents against obligate anaerobic bacteria must be tested under strict anaerobic conditions to accurately reflect their activity in the low-oxygen or oxygen-free environments where these bacteria naturally occur. Standard aerobic testing methods are not suitable for evaluating activity against obligate anaerobes, as these organisms are rapidly killed by exposure to oxygen.

A person skilled in the art cannot simply extrapolate antimicrobial efficacy from aerobic bacteria to obligate anaerobic bacteria. Obligate anaerobic bacteria possess unique cellular envelope structures, distinct membrane lipid compositions, and specialized metabolic pathways adapted strictly to oxygen-free environments. Many conventional antimicrobial agents that rely on oxygen-dependent oxidative stress mechanisms, or that target aerobic respiratory pathways, are rendered ineffective under strict anaerobic conditions. Consequently, there exists a technical prejudice in the art against assuming that standard cosmetic preservatives will exhibit adequate minimum inhibitory concentrations (MIC) against obligate anaerobes in their natural, oxygen-depleted habitats (such as deep sebaceous follicles or periodontal pockets).

### SUMMARY OF INVENTION

There remains a need for antimicrobial agents that are specifically effective against obligate anaerobic bacteria and that have been demonstrated to inhibit such bacteria under strict anaerobic conditions that accurately reflect the natural environments where these bacteria thrive.

In particular, there is a need for antimicrobial agents that can effectively target obligate anaerobic bacteria in applications such as: treating acne-prone skin by inhibiting *Cutibacterium acnes* in the anaerobic environment of sebaceous follicles; oral care applications by inhibiting obligate anaerobic bacteria such as *Fusobacterium nucleatum* in the anaerobic environment of periodontal pockets; and controlling malodor by inhibiting obligate anaerobic bacteria on the skin.

Furthermore, there is a need for antimicrobial agents that are effective at low concentrations, compatible with cosmetic and pharmaceutical formulations, suitable for topical application to the skin and oral cavity, and preferably derived from renewable sources.

The problem to be solved by the present invention is the provision of antimicrobial agents that exhibit effective inhibitory activity against obligate anaerobic bacteria under anaerobic conditions, while maintaining compatibility with cosmetic, dermatological, and pharmaceutical formulations.

The present inventors have surprisingly found that certain amphiphilic compounds, including specifically glyceryl ethers, glyceryl esters, sorbitan esters, isosorbide esters, and N-methyl cyclic glucamides, exhibit potent antimicrobial activity against obligate anaerobic bacteria when tested under strict anaerobic conditions.

More specifically, the inventors have determined minimum inhibitory concentration (MIC) values for these compounds against clinically relevant obligate anaerobic bacteria including *Cutibacterium acnes, Fusobacterium nucleatum,* and *Anaerococcus octavius* under strict anaerobic conditions (80% nitrogen, 10% carbon dioxide, 10% hydrogen atmosphere). The MIC values obtained (ranging from 500 to 4000 ppm) demonstrate that these compounds are effective at concentrations suitable for incorporation into cosmetic, dermatological, and pharmaceutical formulations. Optionally, the compounds may be combined with a further antimicrobial agent to provide a complementary broad-spectrum preservation system effective across various oxygen-depleted and oxygen-rich microenvironments.

The compounds of the present invention are advantageously derived from renewable raw materials and can have a high renewable carbon content, providing sustainable and eco-friendly antimicrobial solutions. The compounds are toxicologically acceptable and suitable for daily use in personal care, cosmetic, and pharmaceutical applications.

Accordingly, in a first aspect, the present invention provides a non-therapeutic use of at least one compound (A) selected from: glyceryl ethers, glyceryl esters, sorbitan esters, isosorbide esters, and N-methyl cyclic glucamides according to formula (I), 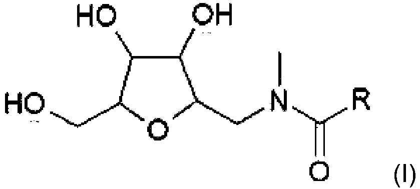
wherein R is selected from saturated hydrocarbon chains having 5 to 23 carbon atoms, unsaturated hydrocarbon chains having 5 to 23 carbon atoms, and
mixtures thereof,
for the inhibition of obligate anaerobic bacteria.

Preferably, R in Formula (I) is selected from saturated hydrocarbon chains having 5 to 17 carbon atoms, unsaturated hydrocarbon chains having 5 to 17 carbon atoms, and mixtures thereof. More preferably, R is selected from saturated or unsaturated hydrocarbon chains having 5 to 13 carbon atoms. Most preferably, R is selected from saturated or unsaturated hydrocarbon chains having 5 to 11 carbon atoms. In a particularly preferred embodiment, R in Formula (I) is selected from saturated or unsaturated hydrocarbon chains having 7 to 9 carbon atoms.

In one preferred embodiment, R in Formula (I) is selected from -(CH₂)₆CH₃, - (CH₂)₈CH₃, -(CH₂)₁₀CH₃, -(CH₂)₁₂CH₃, and mixtures thereof. Particularly preferably, R in Formula (I) is selected from -(CH₂)₆CH₃, -(CH₂)₈CH₃, and mixtures thereof.

Preferably, compound (A) is a mixture of N-methyl cyclic glucamides according to Formula (I) wherein R is -(CH₂)₆CH₃ and compounds wherein R is -(CH₂)₈CH₃, and wherein the weight ratio of said compounds is from 1:9 to 9:1, preferably from 3:7 to 7:3.

When compound (A) is selected from glyceryl ethers, the glyceryl ethers are preferably mono- or diethers of glycerin and one or more C6-C20 fatty alcohols, more preferably C8-C14 fatty alcohols. Particularly preferably, the glyceryl ethers are selected from caprylyl glyceryl ether, ethylhexylglycerin, methylheptylglycerin, and mixtures thereof. Most preferably, the glyceryl ethers are selected from caprylyl glyceryl ether, ethylhexylglycerin, and mixtures thereof.

When compound (A) is selected from glyceryl esters, the glyceryl esters are preferably mono- or diesters of glycerin and one or more C6-C20 fatty acids, more preferably C8-C14 fatty acids. Particularly preferably, the glyceryl esters are selected from glyceryl caprylate, glyceryl caprate, and mixtures thereof.

When compound (A) is selected from sorbitan esters, the sorbitan esters are preferably mono-, di-, or triesters of sorbitan and one or more C6-C20 fatty acids, more preferably mono- or diesters with C8-C14 fatty acids. Particularly preferably, the sorbitan ester is sorbitan caprylate. In certain embodiments, the sorbitan ester has a hydroxyl value of 320 or less, preferably 285 or less, more preferably 245 or less, most preferably 225 or less.

When compound (A) is selected from isosorbide esters, the isosorbide esters are preferably mono- or diesters of isosorbide and one or more C6-C20 fatty acids, more preferably C8-C14 fatty acids. Particularly preferably, the isosorbide ester is isosorbide caprylate. In certain embodiments, the isosorbide ester has a hydroxyl value of 320 or less, preferably 285 or less, more preferably 245 or less, most preferably 225 or less.

In preferred embodiments, the non-therapeutic use comprises the application to the oral cavity, or the topical application of the compound to the skin, particularly for the cosmetic treatment of acne-prone skin or for the control of body malodor. The obligate anaerobic bacteria targeted by this use are preferably selected from the group consisting of *Cutibacterium acnes, Anaerococcus octavius,* and *Fusobacterium nucleatum.*

In one preferred embodiment, the compound (A) is Sorbitan Caprylate, the obligate anaerobic bacterium is *Fusobacterium nucleatum,* and the compound (A) is preferably applied at a concentration of 1000 ppm or less, more preferably 500 ppm or less.

In a second aspect, the present invention provides a compound (A) as defined above for use in a method of treating a medical condition caused by obligate anaerobic bacteria, wherein the treatment comprises inhibiting said obligate anaerobic bacteria.

In preferred embodiments, the medical condition is an infection or inflammatory condition of the skin or the oral cavity. Particularly preferably, the medical condition is acne vulgaris caused by *Cutibacterium acnes* or periodontal disease caused by *Fusobacterium nucleatum.*

In a particularly preferred embodiment, the treatment comprises inhibiting *Cutibacterium acnes,* and the compound (A) is administered in a composition at a concentration of 0.01 wt.-% to 2.0 wt.-%, based on the total weight of the composition.

In a third aspect, the present invention provides a composition for the inhibition of obligate anaerobic bacteria, comprising:
(i) from 0.01 wt.-% to 10.0 wt.-%, based on the total weight of the composition, of at least one compound (A) as defined above; and
(ii) a cosmetically, dermatologically, or pharmaceutically acceptable carrier.

In certain preferred embodiments, the composition comprises from 0.01 wt.-% to 2.0 wt.-% of compound (A), based on the total weight of the composition. Within this range, the composition preferably comprises from 0.05 wt.-% to 1.5 wt.-%, more preferably from 0.1 wt.-% to 1.0 wt.-%, of compound (A), based on the total weight of the composition.

The composition typically has a pH in the range of 2 to 11, preferably 4 to 8, more preferably 4.5 to 7.5, particularly preferably 5 to 7.

Optionally, and advantageously, the composition further comprises a secondary antimicrobial agent. Any known antimicrobial agent in the art can be used. For example, the secondary antimicrobial agent can be selected from phenoxyethanol, benzyl alcohol, benzoic acid and its salts, caprylhydroxamic acid, piroctone olamine, 1,2-octanediol, salicylic acid and its salts, ethylhexylglycerin, triclosan, chlorphenesin, farnesol, C12-15 alkyl benzoate, sodium usnate, silvercitrat, zinc sulfat, zinc ricinoleate, zinc lactate, zinc gluconate, cetrimonium chlorid, hydroxyacetophenone, glyceryl caprylate and mixtures thereof.

In certain preferred embodiments, the composition comprises a synergistic combination of: (a) at least one compound (A) as defined above; and (b) at least one secondary antimicrobial agent; wherein the combination exhibits enhanced antimicrobial activity against both obligate anaerobic bacteria and aerobic bacteria compared to the individual components alone.

The composition may be formulated as a gel, cream, lotion, serum, or cleanser for topical application to the skin; a toothpaste, mouthwash, oral gel, tooth powder, or chewing gum for application to the oral cavity; or a deodorant or antiperspirant for application to the skin. In certain preferred embodiments, the compound (A) incorporated into these formulations is selected from Sorbitan Caprylate, Caprylyl Glyceryl Ether, Ethylhexylglycerin, and N-methyl cyclic glucamides according to formula (I).

In a fourth aspect, the present invention provides an *in vitro* or *ex vivo* method for inhibiting the growth of obligate anaerobic bacteria, comprising the step of contacting said obligate anaerobic bacteria with a composition comprising from 0.01 wt.-% to 2.0 wt.-% of at least one compound (A) as defined above.

The present invention further provides a method of treating a medical condition caused by obligate anaerobic bacteria, comprising administering to a subject an effective amount of compound (A).

Where the medical condition is a skin condition, such as acne vulgaris, the method comprises topically applying a composition comprising 0.01 wt.-% to 2.0 wt.-% of compound (A) to the skin of the subject, preferably to the affected area.

Where the medical condition is a condition of the oral cavity, such as periodontal disease, the method comprises administering a composition comprising 0.01 wt.-% to 2.0 wt.-% of compound (A) to the oral cavity, preferably by rinsing the oral cavity or applying the composition to the gingiva.

In all such methods, the composition is preferably administered one or more times daily.

The present invention provides several significant advantages over the prior art.

Firstly, the invention demonstrates that the compounds of the invention exhibit antimicrobial activity against obligate anaerobic bacteria when tested under strict anaerobic conditions that accurately reflect the natural environments where these bacteria thrive, such as sebaceous follicles, periodontal pockets, and skin surfaces. Prior art documents have not disclosed or demonstrated such activity under anaerobic conditions.

Secondly, the invention provides specific MIC values for these compounds against clinically relevant obligate anaerobes, ranging from 500 to 4000 ppm. This enables precise formulation for effective antimicrobial activity in cosmetic, dermatological, and pharmaceutical applications.

Thirdly, the compounds exhibit excellent formulation compatibility and can be readily formulated into a wide variety of personal care, cosmetic, and dermatological compositions. The compounds are water-soluble or readily dispersible in aqueous systems, stable across a wide pH range, and compatible with conventional cosmetic and pharmaceutical ingredients. Furthermore, when optionally combined with secondary antimicrobial agents that primarily target aerobic organisms and fungi, the compounds of the invention provide a complementary, highly effective broad-spectrum antimicrobial protection system for such complex formulations.

Fourthly, the compounds are derivable from renewable raw materials and have a high renewable carbon content, providing sustainable and eco-friendly antimicrobial solutions. The compounds are toxicologically acceptable and suitable for daily use in personal care applications.

### Examples

Hereinafter, the present invention is described in more detail and specifically with reference to the Examples, which however are not intended to limit the present invention.

### Materials

Velsan^{®} Flex is commercially available from Clariant. Chemical name: Capryloyl/Caproyl Anhydro Methyl Glucamide (and) Water, active content: 70 wt.-%. Capryloyl/Caproyl Anhydro Methyl Glucamide is a mixture of compounds of Formula (I) wherein R is -(CH₂)₈CH₃ and compounds of Formula (I) wherein R is - (CH₂)₆CH₃.

Velsan^{®} SC is commercially available from Clariant. Chemical name: Sorbitan caprylate.

Velsan^{®} CGE is commercially available from Clariant. Chemical name: Caprylyl Glyceryl Ether.

### Example 1 Antimicrobial Activity Against Obligate Anaerobic Bacteria

The antimicrobial properties of Velsan Flex and Velsan SC against anaerobic bacteria were investigated:
Velsan Flex was dissolved in deionized water and Velsan SC was dissolved in dimethyl sulfoxide (DMSO, maximum 50% of flask volume). Each test substance was added in different concentrations to liquid Supplemented Brucella Blood Agar at 50°C. The agar was supplemented with 5% v/v defibrinated sheep blood, hemin stock solution (0.1 ml per 100 ml agar), and vitamin K1 solution (0.1 ml per 100 ml agar), and was buffered to pH 7.0 (+/- 0.1). Final concentrations in the agar plates ranged from 100 to 20,000 ppm (100, 200, 300, 500, 1,000, 2,000, 4,000, 6,000, 8,000, 10,000, and 20,000 ppm).

Each solution was poured into a petri-dish and equilibrated overnight in an anaerobic atmosphere (80% nitrogen, 10% carbon dioxide, 10% hydrogen) at 35°C (+/- 1°C). The plates were then inoculated with standardized suspensions (0.5 McFarland Standard, approximately 1×10⁸ CFU/ml) of anaerobic bacteria using an automatic multipoint inoculator delivering approximately 2 µl per inoculation spot. The test strains included *Fusobacterium nucleatum* DSM 15643, *Cutibacterium acnes* NCTC 10390, and *Anaerococcus octavius* NCTC 9810.

The inoculated plates were incubated for 42-48 hours at 35°C (+/- 1°C) under anaerobic conditions. The minimum inhibitory concentration (MIC) is the lowest concentration of the respective composition that completely inhibits the visible growth of the respective microorganism, disregarding single colonies or faint haze from the original inoculum. Control plates containing a final DMSO concentration of 5% v/v (the same as that included in the highest concentration of DMSO dissolved samples) were included and did not show inhibition of any bacterial strain.

The results are shown in the following Table:

| | Minimum Inhibitory Concentration [ppm] | |
|---|---|---|
| Organisms: | Velsan Flex (70% active solution of Capryloyl/Caproyl Anhydro Methyl Glucamide) | Velsan SC (Sorbitan Caprylate) |
| *Fusobacterium nucleatum* DSM 15643 / ATCC 25586 | 2000 | 500 |
| *Cutibacterium acnes* NCTC 10390 | 2000 | 4000 |
| *Anaerococcus Octavius* NCTC 9810 | 4000 | 4000 |

The MIC values obtained in this study (500-4000 ppm, corresponding to 0.05-0.4 wt.-%) are within the typical concentration range for antimicrobial agents used in cosmetic and personal care formulations.

Antimicrobial preservatives and cosmetic actives commonly used in personal care products typically exhibit MIC values ranging from 100 to 10,000 ppm depending on the target organism, test conditions, and compound class. For example, commonly used antimicrobial agents such as parabens, phenoxyethanol, and organic acids typically show MIC values in the range of 100-5000 ppm against various bacteria under aerobic test conditions.

Obligate anaerobic bacteria are generally more challenging to inhibit than aerobic or facultative anaerobic bacteria due to their specialized metabolic pathways, unique cell wall structures, and adaptation to low-oxygen environments. The MIC values obtained under strict anaerobic conditions in the present study therefore represent effective antimicrobial activity against these specialized organisms.

The demonstrated MIC values of 500-4000 ppm are well within the practical concentration ranges used in cosmetic formulations (typically 0.01-2.0 wt.-%, corresponding to 100-20,000 ppm), providing a comfortable safety margin for formulation development. The formulation examples provided herein (Formulation Examples 1-9) demonstrate that these compounds can be successfully incorporated into various product formats at concentrations of 0.5-2.0 wt.-%, which are at or above the MIC values and therefore provide effective antimicrobial protection against obligate anaerobic bacteria in finished products.

*Cutibacterium acnes* (formerly *Propionibacterium acnes)* is a key causative agent in the development of acne vulgaris. The demonstrated inhibition of C. *acnes* at concentrations of 2000 ppm for Velsan Flex and 4000 ppm for Velsan SC indicates that both compositions are effective as anti-acne actives suitable for incorporation into topical skincare formulations.

*Fusobacterium nucleatum* is an anaerobic bacterium associated with periodontal disease, halitosis, and other oral infections. The effective inhibition of F. *nucleatum* at 2000 ppm for Velsan Flex and notably at 500 ppm for Velsan SC under strict anaerobic conditions demonstrates potent antimicrobial activity. The present invention provides specific MIC values for these compounds against F. *nucleatum* determined under strict anaerobic culture conditions, which more accurately reflects the anaerobic environment of periodontal pockets and other oral sites where obligate anaerobes thrive, and indicates suitability for applications targeting obligate anaerobic bacteria in various environments including the oral cavity.

*Anaerococcus* octavius is an obligate anaerobic bacterium that is part of the skin microbiome and contributes to malodor formation. The inhibition of A. *octavius* at 4000 ppm for both Velsan Flex and Velsan SC under anaerobic conditions demonstrates antimicrobial activity against this organism and indicates suitability for applications targeting obligate anaerobic bacteria on the skin.

These results confirm that Velsan Flex and Velsan SC are effective antimicrobial agents against clinically relevant obligate anaerobic bacteria when tested under strict anaerobic conditions, and are therefore suitable for use in personal care, cosmetic, and pharmaceutical formulations targeting obligate anaerobic bacteria.

### Additional Compounds and Prophetic Examples

While Example 1 demonstrates antimicrobial activity for Velsan SC and Velsan Flex through MIC experimental testing, other compounds within the scope of Formula (I) are expected to exhibit similar antimicrobial activity against obligate anaerobic bacteria based on their structural similarity and known antimicrobial properties.

In particular, Caprylyl Glyceryl Ether (Velsan CGE), which is a glyceryl ether containing a C8 alkyl chain (caprylyl group) similar to the caprylyl moiety in Sorbitan Caprylate, is expected to exhibit antimicrobial activity against obligate anaerobic bacteria. Based on the structure-activity relationships observed for the tested compounds, and considering that both Sorbitan Caprylate and Caprylyl Glyceryl Ether share the same C8 alkyl chain length and amphiphilic character, Caprylyl Glyceryl Ether is expected to exhibit MIC values in the range of 500 to 4000 ppm against obligate anaerobic bacteria such as *Cutibacterium acnes, Anaerococcus octavius,* and *Fusobacterium nucleatum* when tested under the same anaerobic conditions described in Example 1.

The following formulation examples demonstrate the incorporation of Sorbitan Caprylate, Capryloyl/Caproyl Anhydro Methyl Glucamide, and Caprylyl Glyceryl Ether into various personal care compositions suitable for inhibiting obligate anaerobic bacteria. The formulations containing Caprylyl Glyceryl Ether are prophetic examples based on the expected antimicrobial activity of this compound.

### FORMULATION EXAMPLES

The following formulation examples demonstrate the incorporation of the antimicrobial compounds of the invention into various personal care and cosmetic compositions suitable for inhibiting obligate anaerobic bacteria. Unless otherwise specified, all amounts in the formulation examples are given in weight percent (wt.-%) based on the total weight of the composition.

In the tables below:
"q.s. to 100" indicates that water is added in sufficient quantity to bring the total composition to 100 wt.-%;
"-" or "x" indicates that the ingredient is not included in that particular variant; and
"q.s." indicates that the ingredient is added in sufficient quantity to achieve the specified property (e.g., "q.s. pH 6.0")

### Formulation Example 1: Mouthwash Formulations

| | **Example** | | |
|---|---|---|---|
| **Ingredient** | 1a | 1b | 1c |
| Water | q.s. to 100 | q.s. to 100 | q.s. to 100 |
| Sorbitol | 20 | 20 | 20 |
| Glycerin | 10 | 10 | 10 |
| Sodium Saccharin | 0.06 | 0.06 | 0.06 |
| Velsan Flex (Capryloyl/Caproyl Anhydro Methyl Glucamide (and) Water) | 2 | - | - |
| Velsan SC (Sorbitan Caprylate) | - | 2 | - |
| Velsan CGE (Caprylyl glyceryl ether) | - | - | 2 |
| Genapol C 100 (Coceth-10) | 0.5 | 0.5 | 0.5 |
| Flavor | 0.5 | 0.5 | 0.5 |
| Nipaguard SCA (Sorbitan Caprylate (and) Benzyl Alcohol) | 0.8 | 0.8 | 0.8 |
| Herbex Propolis Extract (Butylene Glycol, Water (and) Propolis Extract) | 0.8 | 0.8 | 0.8 |
| HerbEx Green Tea (Butylene Glycol, Water, Camellia Sinensis Leaf Extract) | 1 | 1 | 1 |
| Cooling Agent | 0.5 | 0.5 | 0.5 |

### Formulation Example 2: Toothpaste formulations

| | **Example** | | | | | | |
|---|---|---|---|---|---|---|---|
| **Ingredient** | 2a | 2b | 2c | 2d | 2e | 2f | 2g |
| | Sensitive Teeth | Bleeding Gums | Gingival Re-session | Children's | Whitening | Plaque & Tarter | Daily Use |
| Water | 21.00 | 21.00 | 21.00 | 21.00 | 21.00 | 21.00 | 21.00 |
| Tetrasodium Pyrophosphate | 0.05 | 0.05 | 0.05 | 0.05 | 0.05 | 0.05 | 0.05 |
| Laponite XLS (Sodium Magnesium Silicate) | 3.00 | 3.00 | 3.00 | 3.00 | 3.00 | 3.00 | 3.00 |
| Sorbitol | 20.00 | 20.00 | 20.00 | 20.00 | 18.52 | 18.52 | 18.50 |
| Xanthan Gum | 0.30 | 0.30 | 0.30 | 0.30 | 0.30 | 0.30 | 0.30 |
| PEG 400 (PEG-8) | 2.00 | 2.00 | 2.00 | 2.00 | 2.00 | 2.00 | 2.00 |
| Glycerin | 23.12 | 23.12 | 23.12 | 23.35 | 18.00 | 18.00 | 18.00 |
| Xylitol | 9.55 | 9.55 | 9.55 | 9.55 | 9.55 | 9.55 | 9.55 |
| Sodium Saccharin | 0.30 | 0.30 | 0.30 | 0.30 | 0.30 | 0.30 | 0.30 |
| Sodium Fluoride | 0.23 | 0.23 | 0.23 | x | 0.23 | 0.23 | 0.23 |
| Velsan Flex (Capryloyl/Caproyl Anhydro Methyl Glucamide (and) Water) | 1.10 | 1.10 | 1.10 | 1.10 | 1.10 | 1.10 | 1.10 |
| Aristoflex AVS (Sodium Acryloyldimethyltaurate/VP Crosspolymer) | 0.40 | 0.40 | 0.40 | 0.40 | 0.40 | 0.40 | 0.40 |
| Zeodent 113 (Hydrated Silica) | 7.00 | 7.00 | 7.00 | 7.00 | 7.00 | 7.00 | 7.00 |
| Zeodent 163 (Hydrated Silica) | 9.00 | 9.00 | 9.00 | 9.00 | 9.00 | 9.00 | 9.00 |
| Cornmint (Mint Aroma) | 0.70 | 0.70 | 0.70 | x | 0.70 | 0.70 | 0.70 |
| Titanium Oxide | 0.10 | 0.10 | 0.10 | 0.10 | 0.20 | 0.20 | 0.20 |
| Glucotain Plus (Capryloyl/Caproyl Methyl Glucamide (and) | 2.50 | 2.50 | 2.50 | 2.50 | 4.00 | 4.00 | 4.00 |
| Lauroyl/Myristoyl Methyl Glucamide) | | | | | | | |
| Allura Red (1% Solution) (E129) | x | x | x | 0.02 | x | x | x |
| Strawberry Flavoring | x | x | x | 0.68 | x | x | x |
| Zeodent 124 (Hydrated Silica) | x | x | x | x | 5.00 | 5.00 | 5.00 |
| Brilliant Blue (1% Solution) (Blue 1) | x | x | x | x | x | x | 0.02 |
| TOTAL | 100 | 100 | 100 | 100 | 100 | 100 | 100 |

### Formulation Example 3: Toothpaste Formulations

| | **Example** | | | | | | |
|---|---|---|---|---|---|---|---|
| **Ingredient** | 3a | 3b | 3c | 3d | 3e | 3f | 3g |
| | Sensitive Teeth | Bleeding Gums | Gingival Re-session | Children's | Whitening | Plaque & Tarter | Daily Use |
| Water | 21.00 | 21.00 | 21.00 | 21.00 | 21.00 | 21.00 | 21.00 |
| Tetrasodium Pyrophosphate | 0.05 | 0.05 | 0.05 | 0.05 | 0.05 | 0.05 | 0.05 |
| Laponite XLS (Sodium Magnesium Silicate) | 3.00 | 3.00 | 3.00 | 3.00 | 3.00 | 3.00 | 3.00 |
| Sorbitol | 20.00 | 20.00 | 20.00 | 20.00 | 18.52 | 18.52 | 18.50 |
| Xanthan Gum | 0.30 | 0.30 | 0.30 | 0.30 | 0.30 | 0.30 | 0.30 |
| PEG 400 (PEG-8) | 2.00 | 2.00 | 2.00 | 2.00 | 2.00 | 2.00 | 2.00 |
| Glycerin | 23.12 | 23.12 | 23.12 | 23.35 | 18.00 | 18.00 | 18.00 |
| Xylitol | 9.55 | 9.55 | 9.55 | 9.55 | 9.55 | 9.55 | 9.55 |
| Sodium Saccharin | 0.30 | 0.30 | 0.30 | 0.30 | 0.30 | 0.30 | 0.30 |
| Sodium Fluoride | 0.23 | 0.23 | 0.23 | x | 0.23 | 0.23 | 0.23 |
| Velsan CGE (Caprylyl Glyceryl Ether) | 0.75 | 0.75 | 0.75 | 0.75 | 0.75 | 0.75 | 0.75 |
| Aristoflex AVS (Sodium Acryloyldimethyltaurate/VP Crosspolymer) | 0.40 | 0.40 | 0.40 | 0.40 | 0.40 | 0.40 | 0.40 |
| Zeodent 113 (Hydrated Silica) | 7.00 | 7.00 | 7.00 | 7.00 | 7.00 | 7.00 | 7.00 |
| Zeodent 163 (Hydrated Silica) | 9.00 | 9.00 | 9.00 | 9.00 | 9.00 | 9.00 | 9.00 |
| Cornmint (Mint Aroma) | 0.70 | 0.70 | 0.70 | x | 0.70 | 0.70 | 0.70 |
| Titanium Oxide | 0.10 | 0.10 | 0.10 | 0.10 | 0.20 | 0.20 | 0.20 |
| Glucotain Plus (Capryloyl/Caproyl Methyl Glucamide (and) Lauroyl/Myristoyl Methyl Glucamide) | 2.50 | 2.50 | 2.50 | 2.50 | 4.00 | 4.00 | 4.00 |
| Allura Red (1% Solution) (E129) | x | x | x | 0.02 | x | x | x |
| Strawberry Flavoring | x | x | x | 0.68 | x | x | x |
| Zeodent 124 (Hydrated Silica) | x | x | x | x | 5.00 | 5.00 | 5.00 |
| Brilliant Blue (1% Solution) (Blue 1) | x | x | x | x | x | x | 0.02 |
| TOTAL | 100 | 100 | 100 | 100 | 100 | 100 | 100 |

### Formulation Example 4: Toothpaste Formulations

| | | | | | | | **Example** |
|---|---|---|---|---|---|---|---|
| **Ingredient** | 4a | 4b | 4c | 4d | 4e | 4f | 4g |
| | Sensitive Teeth | Bleeding Gums | Gingival Re-session | Children's | Whitening | Plaque & Tarter | Daily Use |
| Water | 21.00 | 21.00 | 21.00 | 21.00 | 21.00 | 21.00 | 21.00 |
| Tetrasodium Pyrophosphate | 0.05 | 0.05 | 0.05 | 0.05 | 0.05 | 0.05 | 0.05 |
| Laponite XLS (Sodium Magnesium Silicate) | 3.00 | 3.00 | 3.00 | 3.00 | 3.00 | 3.00 | 3.00 |
| Sorbitol | 20.00 | 20.00 | 20.00 | 20.00 | 18.52 | 18.52 | 18.50 |
| Xanthan Gum | 0.30 | 0.30 | 0.30 | 0.30 | 0.30 | 0.30 | 0.30 |
| PEG 400 (PEG-8) | 2.00 | 2.00 | 2.00 | 2.00 | 2.00 | 2.00 | 2.00 |
| Glycerin | 23.12 | 23.12 | 23.12 | 23.35 | 18.00 | 18.00 | 18.00 |
| Xylitol | 9.55 | 9.55 | 9.55 | 9.55 | 9.55 | 9.55 | 9.55 |
| Sodium Saccharin | 0.30 | 0.30 | 0.30 | 0.30 | 0.30 | 0.30 | 0.30 |
| Sodium Fluoride | 0.23 | 0.23 | 0.23 | x | 0.23 | 0.23 | 0.23 |
| Velsan SC (Sorbitan Caprylate) | 0.75 | 0.75 | 0.75 | 0.75 | 0.75 | 0.75 | 0.75 |
| Aristoflex AVS (Sodium AcryloyldimethyltaurateNP Crosspolymer) | 0.40 | 0.40 | 0.40 | 0.40 | 0.40 | 0.40 | 0.40 |
| Zeodent 113 (Hydrated Silica) | 7.00 | 7.00 | 7.00 | 7.00 | 7.00 | 7.00 | 7.00 |
| Zeodent 163 (Hydrated Silica) | 9.00 | 9.00 | 9.00 | 9.00 | 9.00 | 9.00 | 9.00 |
| Cornmint (Mint Aroma) | 0.70 | 0.70 | 0.70 | x | 0.70 | 0.70 | 0.70 |
| Titanium Oxide | 0.10 | 0.10 | 0.10 | 0.10 | 0.20 | 0.20 | 0.20 |
| Glucotain Plus (Capryloyl/Caproyl Methyl Glucamide (and) Lauroyl/Myristoyl Methyl Glucamide) | 2.50 | 2.50 | 2.50 | 2.50 | 4.00 | 4.00 | 4.00 |
| Allura Red (1% Solution) (E129) | x | x | x | 0.02 | x | x | x |
| Strawberry Flavoring | x | x | x | 0.68 | x | x | x |
| Zeodent 124 (Hydrated Silica) | x | x | x | x | 5.00 | 5.00 | 5.00 |
| Brilliant Blue (1% Solution) (Blue 1) | x | x | x | x | x | x | 0.02 |
| TOTAL | 100 | 100 | 100 | 100 | 100 | 100 | 100 |

### Formulation Example 5: Deodorant Formulations

| | **Example** | | |
|---|---|---|---|
| **Ingredient** | 5a | 5b | 5c |
| Velsan Flex (Capryloyl/Caproyl Anhydro Methyl Glucamide (and) Water) | 1 | - | - |
| Velsan SC (Sorbitan Caprylate) | - | 0.5 | - |
| Velsan CGE (Caprylyl glyceryl ether) | - | - | 0.5 |
| Xanthan Gum | 0.5 | 0.5 | 0.5 |
| Aristoflex^{®} Silk (Sodium Polyacryloyldimethyl Taurate) | 0.3 | 0.3 | 0.3 |
| GlucoTain Sense (Sunfloweroyl Methyl Glucamide) | 2.0 | 2.0 | 2.0 |
| Fragrance | 1 | 1 | 1 |
| Magnesium Hydroxide (and) Magnesium Hydroxide Carbonate | 7.50 | 7.50 | 7.50 |
| Water | q.s. to 100 | q.s. to 100 | q.s. to 100 |

### Formulation Example 6: Deodorant Stick

| | **Example** | | |
|---|---|---|---|
| **Ingredient** | 6a | 6b | 6c |
| Magnesium Hydroxide (and) Magnesium Hydroxide Carbonate | 20.0 | 20.0 | 20.0 |
| Tapioca Starch | 9.0 | 9.0 | 9.0 |
| Distarch Phosphate | 10.0 | 10.0 | 10.0 |
| Citrus Lemon Peel Oil | 0.5 | 0.5 | 0.5 |
| Tocopherol (and) Helianthus Annuus (Sunflower) Seed Oil | 1.0 | 1.0 | 1.0 |
| Dodecane | 15.0 | 15.0 | 15.0 |
| Citrus Aurantifolia (Lime) Seed Oil, Hydrogenated Vegetable Oil | 5.0 | 5.0 | 5.0 |
| Olea Europaea (Olive) Fruit Oil | 4.5 | 4.5 | 4.5 |
| Squalane | 3.0 | 3.0 | 3.0 |
| Hydrogenated Vegetable Oil | 5.0 | 5.0 | 5.0 |
| Velsan Flex (Capryloyl/Caproyl Anhydro Methyl Glucamide (and) Water) | | | 1.0 |
| Velsan SC (Sorbitan Caprylate) | | 1.0 | |
| Velsan CGE (Caprylyl glyceryl ether) | 1.0 | | |
| Butylene Glycol, Aqua, Centella Asiatica Extract, PolygonumCuspidatum Root Extract, Scutellaria Baicalensis Root Extract | 1.0 | 1.0 | 1.0 |
| Ozokerite | 13.0 | 13.0 | 13.0 |
| Cetearyl Alcohol | 10.0 | 10.0 | 10.0 |

### Formulation Example 7: Anti-Acne formulations (Ethanol containing)

| | **Example** | | |
|---|---|---|---|
| **Ingredient** | 7a | 7b | 7c |
| Velsan Flex (Capryloyl/Caproyl Anhydro Methyl Glucamide (and) Water) | 1 | | |
| Velsan SC (Sorbitan Caprylate) | | 1 | |
| Velsan CGE (Caprylyl glyceryl ether) | | | 1 |
| Ethanol | 25.0 | 25.0 | 25.0 |
| Propylene glycol | 25.0 | 25.0 | 25.0 |
| Fragrance | 0.2 | 0.2 | 0.2 |
| Preservative | 1 | 1 | 1 |
| Aristoflex HMB (Ammonium Acryloyl Dimethyl Taurate/Beheneth-25) | 1.3 | 1.3 | 1.3 |
| Allantoin | 0.1 | 0.1 | 0.1 |
| Water | q.s. to 100 | q.s. to 100 | q.s. to 100 |

### Formulation Example 8: Facial Cleanser (Anti-Acne)

| | **Example** | | |
|---|---|---|---|
| **Ingredient** | 8a | 8b | 8c |
| Water | q.s. to 100 | q.s. to 100 | q.s. to 100 |
| Glycerin (85%) | 3.00 | 3.00 | 3.00 |
| Capixyl (Butylene Glycol (and) Water (and) Dextran (and) Acetyl Tetrapeptide-3 (and) Trifolium Pratense (Clover) Flower Extract) | 2.00 | 2.00 | 2.00 |
| Benzyl alcohol | 0.30 | 0.30 | 0.30 |
| Aristoflex Velvet (Polyacrylate Crosspolymer-11) | 1.00 | 1.00 | 1.00 |
| Velsan Flex (Capryloyl/Caproyl Anhydro Methyl Glucamide (and) Water) | 1.5 | 0 | 0 |
| Velsan SC (Sorbitan Caprylate) | 0 | 1.5 | 0 |
| Velsan CGE (Caprylyl glyceryl ether) | 0 | 0 | 1.5 |
| Fragrance | 0.1 | 0.1 | 0.1 |
| GlucoTain liquiFlex (Lauroyl/Myristoyl Methyl Glucamide (and) Coco-Betaine) | 1.50 | 1.50 | 1.50 |
| Sodium Hydroxide/Citric Acid Solution | q.s. pH 6.0-6.5 | q.s. pH 6.0-6.5 | q.s. pH 6.0-6.5 |

### Formulation Example 9: Moisturizing Anti-Acne Gel

| | **Example** | | |
|---|---|---|---|
| **Ingredient** | 9a | 9b | 9c |
| Water | q.s. to 100 | q.s. to 100 | q.s. to 100 |
| Propylene Glycol | 4.00 | 4.00 | 4.00 |
| Allantoin | 0.50 | 0.50 | 0.50 |
| Sorbitol | 1.00 | 1.00 | 1.00 |
| Velsan Flex (Capryloyl/Caproyl Anhydro Methyl Glucamide (and) Water) | 2 | 0 | 0 |
| Velsan SC (Sorbitan Caprylate) | 0 | 2 | 0 |
| Velsan CGE (Caprylyl glyceryl ether) | 0 | 0 | 2 |
| Phenoxyethanol | 0.5 | 0.5 | 0.5 |
| Carbomer | 0.80 | 0.80 | 0.80 |
| Sodium Hydroxide | q.s. pH 5.8 | q.s. pH 5.8 | q.s. pH 5.8 |

## Claims

1. Non-therapeutic use of at least one compound (A) selected from: glyceryl ethers, glyceryl esters, sorbitan esters, isosorbide esters, and N-methyl cyclic glucamides according to formula (I), 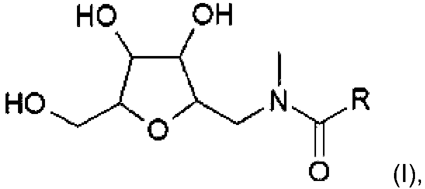
wherein R is selected from saturated hydrocarbon chains having 5 to 23 carbon atoms, unsaturated hydrocarbon chains having 5 to 23 carbon atoms, and mixtures thereof,
for the inhibition of obligate anaerobic bacteria.

2. The non-therapeutic use according to claim 1, wherein the use comprises the application to the oral cavity.

3. The non-therapeutic use according to claim 1, wherein the use comprises the topical application of the compound to the skin, preferably for the cosmetic treatment of acne-prone skin or for the control of body malodor.

4. The non-therapeutic use according to any one of the preceding claims, wherein the obligate anaerobic bacteria are selected from the group consisting of *Cutibacterium acnes, Anaerococcus octavius,* and *Fusobacterium nucleatum.*

5. The non-therapeutic use according to any one of the preceding claims, wherein compound (A) is selected from the group consisting of:
(a) mono- or diethers of glycerin and one or more C8-C14 fatty alcohols, preferably caprylyl glyceryl ether or ethylhexylglycerin; and
(b) mono- or diesters of sorbitan or isosorbide and one or more C8-C14 fatty acids, preferably sorbitan caprylate or isosorbide caprylate.

6. The non-therapeutic use according to any one of claims 1 to 4, wherein compound (A) is a mixture of N-methyl cyclic glucamides according to Formula (I) wherein R is -(CH₂)₆CH₃ and compounds wherein R is -(CH₂)₈CH₃, and wherein the weight ratio of said compounds is from 1:9 to 9:1, preferably from 3:7 to 7:3.

7. The non-therapeutic use according to any one of the preceding claims, wherein compound (A) is present in a composition at a concentration of from 0.01 wt.-% to 10.0 wt.-%, preferably from 0.5 wt.-% to 2.0 wt.-%, based on the total weight of the composition.

8. The non-therapeutic use according to any one of the preceding claims, wherein the compound (A) is Sorbitan Caprylate, the obligate anaerobic bacterium is *Fusobacterium nucleatum,* and wherein the compound (A) is applied at a concentration of preferably 1000 ppm or less, more preferably 500 ppm or less.

9. A compound (A) as defined in claim 1, 5 or 6 for use in a method of treating a medical condition caused by obligate anaerobic bacteria, wherein the treatment comprises inhibiting said obligate anaerobic bacteria.

10. The compound for use according to claim 9, wherein the medical condition is acne vulgaris caused by *Cutibacterium acnes* or periodontal disease caused by *Fusobacterium nucleatum.*

11. A composition for the inhibition of obligate anaerobic bacteria, comprising:
(i) from 0.01 wt.-% to 10.0 wt.-%, preferably from 0.5 wt.-% to 2.0 wt.-%, based on the total weight of the composition, of a compound (A) as defined in claim 1, 5 or 6; and
(ii) a cosmetically, dermatologically, or pharmaceutically acceptable carrier.

12. The composition according to claim 11, further comprising a secondary antimicrobial agent.

13. The composition according to claim 12, wherein the composition is formulated as:
(a) a gel, cream, lotion, serum, or cleanser for topical application to the skin;
(b) a toothpaste, mouthwash, or oral gel for application to the oral cavity; or
(c) a deodorant for application to the skin.

14. The composition according to claim 12 or 13, wherein the compound (A) is selected from Sorbitan Caprylate, Caprylyl Glyceryl Ether, and N-methyl cyclic glucamides according to formula (I).

15. An *in vitro* or *ex vivo* method for inhibiting the growth of obligate anaerobic bacteria, comprising the step of contacting said obligate anaerobic bacteria with a composition comprising from 0.01 wt.-% to 2.0 wt.-% of at least one compound (A) as defined in claim 1, 5 or 6.
